# EUROPÄISCHE PATENTSCHRIFT

(11) **EP 1 367 948 B1**
(45) Veröffentlichungstag und Bekanntmachung des Hinweises auf die Patenterteilung: **01.09.2004**
(21) Anmeldenummer: 02703623.5
(22) Anmeldetag: 07.03.2002
(51) Int. Cl.: A61B 17/28

(54) **MEDIZINISCHES GREIFINSTRUMENT**
MEDICAL GRIPPING INSTRUMENT
INSTRUMENT DE PREHENSION MEDICAL

(30) Priorität: 12.03.2001 DE 10111766
(43) Veröffentlichungstag der Anmeldung: 10.12.2003
(73) Patentinhaber: Karl Storz GmbH & Co. KG, 78532 Tuttlingen (DE)
(72) Erfinder: BACHER, Uwe, 78532 Tuttlingen (DE)
(74) Vertreter: Hofmeister, Frank Horst
(86) Internationale Anmeldenummer: PCT/EP2002/002490
(87) Internationale Veröffentlichungsnummer: WO 2002/071956

(56) Entgegenhaltungen:
- EP-A- 0 588 658
- EP-A- 0 647 433
- EP-A- 0 813 843
- EP-A- 1 066 798
- WO-A-01/19261
- US-A- 5 496 347

## Beschreibung

Die Erfindung betrifft ein medizinisches Instrument mit einem Schaft, an dessen proximalem Ende eine aus zwei Griffteilen bestehende Handhabe angeordnet ist und an dessen distalem Ende ein aus zwei um einen gemeinsamen Drehpunkt gegeneinander verschwenkbaren Maulteilen bestehendes Werkzeug angeordnet ist, wobei das Verschwenken der Maulteile zum Öffnen und Schließen des Werkzeugs über eine Zug-/Druckstange erfolgt, die proximalseitig mit einem verschwenkbar ausgebildeten Griffteil der Handhabe verbunden ist und die distalseitig über jeweils einen Gelenkhebel mit jedem der Maulteile verbunden ist.

Gattungsgemäße medizinische Instrumente werden in der Praxis häufig als Greif-, Halte- und/oder Schneidwerkzeuge eingesetzt. So können die Maulteile Schneiden aufweisen, um Gewebe abzutrennen, oder stumpfe Flächen aufweisen, um beispielsweise abgetrenntes Gewebe zu halten, oder Blutgefäße abzuklemmen.

Diesen medizinischen Instrumenten ist gemeinsam, daß beide Maulteile des am distalen Ende des Schaftes angeordneten Werkzeugs um einen gemeinsamen Drehpunkt verschwenkbar sind. Zum Öffnen und Schließen der Maulteile ist eine Zug-/Druckstange vorgesehen, die mit einem beweglichen Griffteil der Handhabe verbunden ist. Um ausgehend von der einen Zug-/Druckstange zwei Maulteile verschwenken zu können, sind die Maulteile und die Zug-/Druckstange über jeweils einen Gelenkhebel miteinander verbunden, die jeweils einerseits gelenkig mit der Zug-/Druckstange und andererseits gelenkig mit dem jeweiligen Maulteil verbunden sind.

Ein solches medizinisches Instrument ist beispielsweise aus der DE 299 11 011 U bekannt. Bei diesem bekannten medizinischen Instrument liegen die Anlenkpunkte der Gelenkhebel an den jeweiligen Maulteilen weit entfernt von dem gemeinsamen Drehpunkt der Maulteile, damit sich beim Verschieben der Zug-/Druckstange, insbesondere zum Schließen des Werkzeugs, ein Hebelverhältnis einstellt, daß eine große Kraftübertragung ermöglicht. Diese medizinischen Instrumente haben sich in der Praxis bewährt, jedoch weisen sie unter beengten Platzverhältnissen den Nachteil auf, daß die Gelenkhebel beim Öffnen der Maulteile nach außen schwenken und so im Bereich zwischen Zug-/Druckstange und Werkzeug den Durchmesser des Instruments deutlich vergrößern. Dieser Platzbedarf steht aber nicht immer zur Verfügung steht, weshalb diese bekannten Instrumente nicht bei allen Operationen eingesetzt werden können.

Davon ausgehend liegt der Erfindung die **Aufgabe** zugrunde, ein medizinisches Instrument der eingangs genannten Art so auszugestalten, daß dieses auch unter beengten Platzverhältnissen und mit ausreichender Kraftübertragung einstellbar ist.

Die **Lösung** dieser Aufgabenstellung ist erfindungsgemäß dadurch gekennzeichnet, daß die Anlenkpunkte der Gelenkhebel am jeweiligen Maulteil entfernt vom proximalen Ende der Maulteile, nahe am gemeinsamen Drehpunkt der Maulteile angeordnet sind und auch bei maximaler Auslenkung innerhalb des distalseitigen Außendurchmessers des die Zug-/Druckstange unmittelbar umgebenden Schaftes liegen und, daß jeder Gelenkhebel über jeweils nur einen Anlenkpunkt an der Zug-/Druckstange gelagert ist, wobei der Anlenkpunkt des einen Gelenkhebels an der Zug-/Druckstange oberhalb der Mittelachse der Zug-/Druckstange liegt und der Anlenkpunkt des anderen Gelenkhebels an der Zug-/Druckstange unterhalb der Mittelachse der Zug-/Druckstange liegt, und daß das mit den Gelenkhebeln verbundene distale Ende der Zug-/Druckstange zweiarmig ausgebildet ist, wobei die Anlenkpunkte der Gelenkhebel an den vorderen Enden der Arme angeordnet sind und ein Arm fort von der Mittelachse der Zug-/Druckstange nach oben und der andere Arm fort von der Mittelachse der Zug-/Druckstange nach unten abgewinkelt ausgebildet ist.

Durch die Verlagerung der Anlenkpunkte der Gelenkhebel an den Maulteilen hin zum gemeinsamen Drehpunkt der Maulteile wird ein Instrument zur Verfügung gestellt, dessen beide Maulteile sich über eine Zug-/Druckstange verschwenken lassen, das aber auch bei beengtesten Platzverhältnissen einsetzbar ist, da die Anlenkpunkte aufgrund der großen Nähe zum gemeinsamen Drehpunkt auch bei maximaler Auslenkung nicht so weit nach außen verschwenkt werden, daß sie über den vorgegebenen Durchmesser des Schaftes hinausragen. Da sich das Hebelverhältnis und somit die insbesondere beim Schließen des Werkzeugs aufbringbaren Kräfte durch das Verlagern der Anlenkpunkte hin zum gemeinsamen Drehpunkt gegenüber den Instrumenten des Standes der Technik verschlechtert haben, ist der Anlenkpunkt des einen Gelenkhebels an der Zug-/Druckstange oberhalb der Mittelachse der Zug-/Druckstange und der Anlenkpunkt des anderen Gelenkhebels an der Zug-/Druckstange unterhalb der Mittelachse der Zug-/Druckstange angeordnet. Durch dieses Verlagern der Anlenkpunkte zwischen den Gelenkhebeln und der Züg-/Druckstange fort von der Mittelachse kann das für die mögliche Kraftübertragung verantwortliche Hebelverhältnis wieder verbessert werden, so daß auch Schneidvorgänge zuverlässig ausführbar sind.

Durch dieses Aufspreizen des mit den Gelenkhebeln verbundenen distalen Endes der Zug-/Druckstange läßt sich das Hebelverhältnis noch weiter verbessern.

Weitere Merkmale und Vorteile der Erfindung ergeben sich aus der nachfolgenden Beschreibung der zugehörigen Zeichnung, in der ein Ausführungsbeispiel eines erfindungsgemäßen medizinischen Instruments nur beispielhaft schematisch dargestellt ist. In der Zeichnung zeigt:
- Fig. 1: eine Seitenansicht eines erfindungsgemäßen medizinischen Instruments;
- Fig. 2: eine vergrößerte und teilweise geschnittene Darstellung des Details II gemäß Fig. 1, die Maulteile im geschlossenen Zustand darstellend und
- Fig. 3: eine vergrößerte und teilweise geschnittene Darstellung des Details III gemäß Fig. 1, die Maulteile im geöffneten Zustand darstellend.

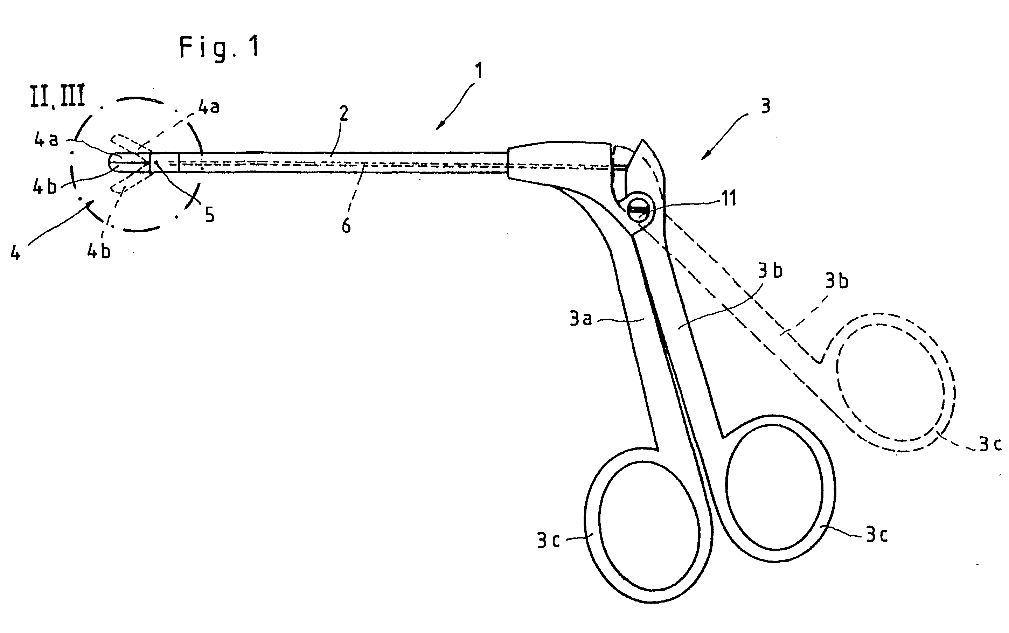

Die Abbildung Fig. 1 zeigt eine Seitenansicht eines medizinischen Instruments, dessen Kraftübertragungsmechanismus vielseitig verwendet werden kann, wie beispielsweise für Stanzen, Scheren, Nadelhalter, Faßinstrumente und dergleichen.

Das dargestellte medizinische Instrument 1 besteht im wesentlichen aus einem hohlen Schaft 2, an dessen proximalem Ende eine Handhabe 3 angeordnet ist, die aus einem starren Griffteil 3a und einem gegenüber dem starren Griffteil 3a verschwenkbaren Griffteil 3b besteht. Am distalen Ende des Schaftes 2 ist ein Werkzeug 4 angeordnet, welches von zwei gegeneinander um einen gemeinsamen Drehpunkt 5 verschwenkbaren Maulteilen 4a und 4b gebildet wird.

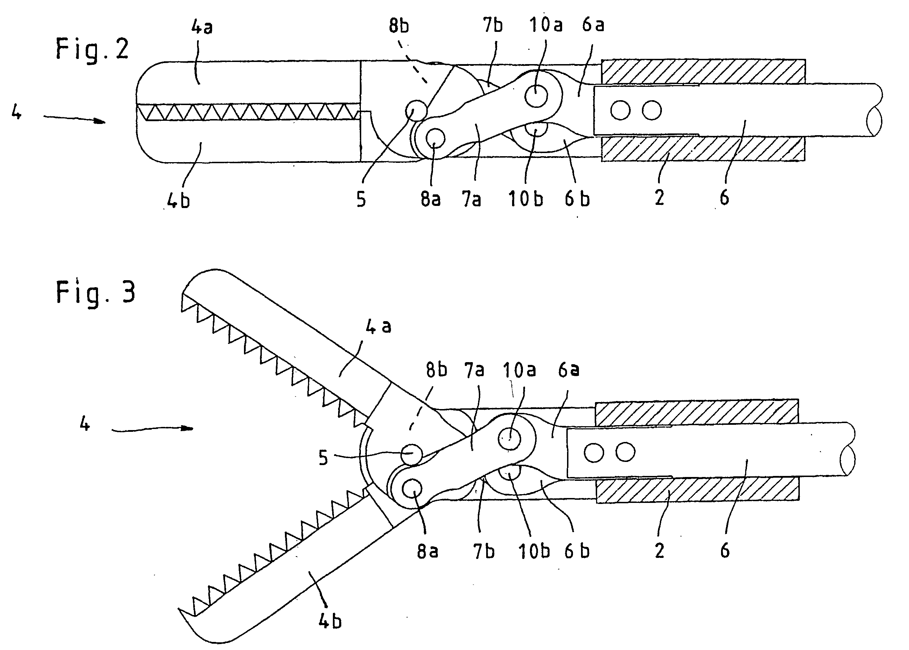

Wie aus den Detailansichten gemäß Fig. 2 und 3 sowie der Komplettansicht gemäß Fig. 1 ersichtlich, sind die Maulteile 4a und 4b des Werkzeugs 4 und das verschwenkbare Griffteil 3b der Handhabe 3 über eine Zug-/Druckstange 6 miteinander so verbunden, daß durch das Verstellen des Griffteils 3b die Maulteile 4a und 4b von der geschlossenen Stellung (durchgezogene Darstellung in Fig. 1 sowie Fig. 2) in die geöffnete Stellung (gestrichelte Darstellung in Fig. 1 sowie Fig. 3) bzw. umgekehrt überführbar sind. Die jeweils zugehörige Stellung des verschwenkbaren Griffteils 3b ist in der Abbildung Fig.1 ebenfalls durchgezogen (für die geschlossene Stellung) und gestrichelt (für die geöffnete Stellung) dargestellt.

Den Detailansichten Fig. 2 und 3 ist weiterhin zu entnehmen, daß die Zug-/Druckstange 6 nicht unmittelbar, sondern unter Zwischenschaltung zweier Gelenkhebel 7a und 7b mit den Maulteilen 4a und 4b verbunden sind. Die Gelenkhebel 7a und 7b ermöglichen es, daß mit nur einer Zug-/Druckstange 6 beide Maulteile 4a und 4b verstellt werden können und auch noch eine ausreichende Kraftübertragung auf das Werkzeug 4, insbesondere beim Schließen der Maulteile 4a und 4b, gewährleistet ist.

Um beim Ankoppeln der Zug-/Druckstange 6 an die Maulteile 4a und 4b des Werkzeugs 4 über die Gelenkhebel 7a und 7b sicherzustellen, daß einerseits über die Maulteile 4a und 4b eine ausreichende Schneid- bzw. Klemmkraft aufbringbar ist und andererseits die Abmessungen des Instruments durch den Hebelmechanismus nicht vergrößert werden, sind zum einen die Gelenkhebel 7a, 7b über Anlenkpunkte 8a und 8b mit den Maulteilen 4a und 4b verbunden, die nahe dem gemeinsamen Drehpunkt 5 der Maulteile 4a, 4b angeordnet sind und ist zum anderen das distalseitige Ende der Zug-/Druckstange 6 zweiarmig mit zwei von der Mittelachse 9 der Zug-/Druckstange 6 fortweisend abgewinkelten Armen 6a und 6b versehen ausgebildet.

Der genaue Aufbau des Hebelmechanismus und die Anordnung der Bauteile zueinander ist den Abbildungen Fig. 2 und 3 zu entnehmen. Die Verbindung der Gelenkhebel 7a und 7b mit den Armen 6a und 6b der Zug-/Druckstange 6 erfolgt über Anlenkpunkte 10a und 10b, die oberhalb und unterhalb der Mittelachse 9 der Zug-/Druckstange 6 angeordnet sind. Durch das Verlagern der Anlenkpunkte 10a und 10b fort von der Mittelachse 9 der Zug-/Druckstange 6 wird das erzielbare Hebelverhältnis wieder verbessert, welches durch die Verlagerung der Anlenkpunkte 8a und 8b hin zum gemeinsamen Drehpunkt 5 der Maulteile 4a, 4b verschlechtert wurde. Dieses Verlagern der Anlenkpunkte 8a, 8b ist aber notwendig, da nur auf diese Weise gewährleistet werden kann, daß die Anlenkpunkte 8a, 8b auch bei maximaler Auslenkung der Maulteile 4a, 4b bzw. der Gelenkhebel 7a, 7b nicht über den Durchmesser des Schaftes 2 hinaustreten.

Das Betätigen des medizinischen Instruments 1 geschieht wie folgt:

Zum sicheren Ergreifen der Griffteile 3a, 3b der Handhabe 3 weisen diese an ihren freien Enden Fingerösen 3c auf. Beim dargestellten Ausführungsbeispiel ist das Griffteil 3b um eine Schwenkachse 11 gegenüber dem anderen, starren Griffteil 3a verschwenkbar.

Durch die Kopplung des verschwenkbaren Griffteits 3b über die Zug-/Druckstange 6 und die Gelenkhebel 7a, 7b mit den verschwenkbaren Maulteilen 4a, 4b des Werkzeugs 4 läßt sich das Werkzeug 4 öffnen und schließen.

Wie aus den Abbildungen Fig. 2 und 3 ersichtlich sind die Anlenkpunkte 8a, 8b und 10a, 10b zum Verbinden der Gelenkhebel 7a und 7b mit den Maulteilen 4a, 4b und den Armen 6a, 6b der Zug-/Druckstange 6 so angeordnet, daß keiner der Anlenkpunkte 8a, 8b oder 10a, 10b beim Verstellen der Maulteile 4a, 4b über den Durchmesser des Schaftes 2 hinaustritt. Ein solchermaßen ausgebildetes medizinisches Instrument 1 ist somit auch unter beengtesten Platzverhältnissen einsetzbar.

### Bezugszeichenliste

- 1: medizinisches Instrument
- 2: Schaft
- 3: Handhabe
- 3a: starres Griffteil
- 3b: verschwenkbares Griffteil
- 3c: Fingeröse
- 4: Werkzeug
- 4a: Maulteil
- 4b: Maulteil
- 5: Drehpunkt
- 6: Zug-/Druckstange
- 6a: Arm
- 6b: Arm
- 7a: Gelenkhebel
- 7b: Gelenkhebel
- 8a: Anlenkpunkt
- 8b: Anlenkpunkt
- 9: Mittelachse
- 10a: Anlenkpunkt
- 10b: Anlenkpunkt
- 11: Schwenkachse

## Patentansprüche

1. Medizinisches Instrument mit einem Schaft (2), an dessen proximalem Ende eine aus zwei Griffteilen (3a, 3b) bestehende Handhabe (3) angeordnet ist und an dessen distalem Ende ein aus zwei um einen gemeinsamen Drehpunkt (5) gegeneinander verschwenkbaren Maulteilen (4a, 4b) bestehendes Werkzeug (4) angeordnet ist, wobei das Verschwenken der Maulteile (4a, 4b) zum Öffnen und Schließen des Werkzeugs (4) über eine Zug-/Druckstange (6) erfolgt, die proximalseitig mit einem verschwenkbar ausgebildeten Griffteil (3b) der Handhabe (3) verbunden ist und die distalseitig über jeweils einen Gelenkhebel (7a, 7b) mit jedem der Maulteile (4a, 4b) verbunden ist,
**dadurch gekennzeichnet,**
**daß** die Anlenkpunkte (8a, 8b) der Gelenkhebel (7a, 7b) am jeweiligen Maulteil (4a, 4b) entfernt vom proximalen Ende der Maulteile (4a, 4b), nahe am gemeinsamen Drehpunkt (5) der Maulteile (4a, 4b) angeordnet sind und auch bei maximaler Auslenkung innerhalb des distalseitigen Außendurchmessers des die Zug-/Druckstange (6) unmittelbar umgebenden Schaftes (2) liegen und, daß jeder Gelenkhebel (7a, 7b) über jeweils nur einen Anlenkpunkt (10a, 10b) an der Zug-/Druckstange (6) gelagert ist, wobei der Anlenkpunkt (10a) des einen Gelenkhebels (7a) an der Zug-/Druckstange (6) oberhalb der Mittelachse (9) der Zug-/Druckstange (6) liegt und der Anlenkpunkt (10b) des anderen Gelenkhebels (7b) an der Zug-/Druckstange (6) unterhalb der Mittelachse (9) der Zug-/Druckstange (6) liegt, und daß das mit den Gelenkhebeln (7a, 7b) verbundene distale Ende der Zug-/Druckstange (6) zweiarmig ausgebildet ist, wobei die Anlenkpunkte (10a, 10b) der Gelenkhebel (7a, 7b) an den vorderen Enden der Arme (6a, 6b) angeordnet sind und ein Arm (6a) fort von der Mittelachse (9) der Zug-/Druckstange (6) nach oben und der andere Arm (6b) fort von der Mittelachse (9) der Zug-/Druckstange (6) nach unten abgewinkelt ausgebildet ist.

## Claims

1. Medical instrument with a shaft (2) at whose proximal end there is a handle (3) consisting of two grip parts (3a, 3b), and at whose distal end there is a tool (4) consisting of two jaw parts (4a, 4b) which can pivot relative to one another about a common pivot point (5), the pivoting of the jaw parts (4a, 4b), for the purpose of opening and closing the tool (4), being effected via a pull/push rod (6) which at the proximal end is connected to a pivotable grip part (3b) of the handle (3) and which at the distal end is connected to each of the jaw parts (4a, 4b) via a respective articulated lever (7a, 7b), **characterized in that** the hinge points (8a, 8b) of the articulated levers (7a, 7b) on the respective jaw part (4a, 4b) are arranged remote from the proximal end of the jaw parts (4a, 4b), near to the common pivot point (5) of the jaw parts (4a, 4b), and, even upon maximum opening, lie inside the distal external diameter of the shaft (2) directly enclosing the pull-push rod (6), **in that** each articulated lever (7a, 7b) is mounted on the pull/push rod (6) via in each case only one hinge point (10a, 10b), the hinge point (10a) of one articulated lever (7a) on the pull/push rod (6) lying above the centre axis (9) of the pull/push rod (6), and the hinge point (10b) of the other articulated lever (7b) on the pull/push rod (6) lying under the centre axis (9) of the pull/push rod (6), and **in that** the distal end of the pull/push rod (6) connected to the articulated levers (7a, 7b) is designed with two arms, the hinge points (10a, 10b) of the articulated levers (7a, 7b) being arranged on the front ends of the arms (6a, 6b) , and one arm (6a) is angled upwards away from the centre axis (9) of the pull/push rod (6) and the other arm (6b) is angled downwards away from the centre axis (9) of the pull/push rod (6).

## Revendications

1. Instrument médical comprenant un corps en tige (2) à l'extrémité proximale duquel est disposé une poignée de manoeuvre (3) constituée de deux pièces de préhension (3a, 3b), et à l'extrémité distale duquel est disposé un outil (4) constitué de deux pièces de mâchoire (4a, 4b) pouvant pivoter l'une par rapport à l'autre autour d'un point de rotation commun (5), le pivotement des pièces de mâchoire (4a, 4b) pour ouvrir et fermer l'outil (4) s'effectuant par l'intermédiaire d'un tirant de traction/compression (6) qui, côté proximal, est relié à une pièce de préhension (3b) de configuration pivotante, de la poignée de manoeuvre (3), et qui, côté distal, est relié à chacune des pièces de mâchoire (4a, 4b) respectivement par un levier d'articulation (7a, 7b), **caractérisé en ce que** les points d'articulation (8a, 8b) des leviers d'articulation (7a, 7b) sur la pièce de mâchoire (4a, 4b) respective, sont disposés de manière éloignée de l'extrémité proximale des pièces de mâchoire (4a, 4b), à proximité du point de rotation commun (5) des pièces de mâchoire (4a, 4b) et se situent, également en cas de déviation maximale, à l'intérieur du diamètre extérieur, côté distal, du corps en tige (2) entourant directement le tirant de traction/compression (6), et **en ce que** chaque levier d'articulation (7a, 7b) n'est monté respectivement que par l'intermédiaire d'un seul point d'articulation (10a, 10b) sur le tirant de traction/compression (6), le point d'articulation (10a) de l'un des leviers d'articulation (7a) sur le tirant de traction/compression (6) se situant au-dessus de l'axe médian (9) du tirant de traction/compression (6), et le point d'articulation (10b) de l'autre levier d'articulation (7b) sur le tirant de traction/compression (6) se situant en-dessous de l'axe médian (9) du tirant de traction/compression (6), et **en ce que** l'extrémité distale du tirant de traction compression (6), reliée aux leviers d'articulation (7a, 7b), est réalisée à deux bras, les points d'articulation (10a, 10b) des leviers d'articulation (7a, 7b) étant disposés aux extrémités avant des bras (6a, 6b), et un bras (6a) étant réalisé de manière coudée vers le haut en partant de l'axe médian (9) du tirant de traction/compression (6) et l'autre bras (6b) de manière coudée vers le bas en partant de l'axe médian (9) du tirant de traction/compression (6).
